# EUROPEAN PATENT APPLICATION

(11) **EP 4 473 931 A1**
(43) Date of publication of application: **11.12.2024**
(21) Application number: 24180708.0
(22) Date of filing: 07.06.2024
(51) Int. Cl.: A61B 90/00, A61C 1/08, B33Y 80/00

(54) **SUPPORT DEVICE FOR A DYNAMIC REFERENCE SENSOR FOR TRACKING IN NAVIGATED MAXILLOFACIAL SURGERY**

(30) Priority: 08.06.2023 IT 202300011781
(71) Applicant: UNIVERSITA' DEGLI STUDI MAGNA GRAECIA DI CATANZARO, 88100 Catanzaro (IT); Arga Medicali S.r.l., 00193 Roma (RM) (IT)
(72) Inventor: CRISTOFARO, MARIA GIULIA, 88100 CATANZARO (CZ) (IT); LAVANO, ANGELO, 88100 CATANZARO (CZ) (IT); SABATINI, UMBERTO, 00069 TREVIGNANO ROMANO (RM) (IT); VELTRI, PIERANGELO, 87036 RENDE (CS) (IT); LA TORRE, DOMENICO, 98121 MESSINA (ME) (IT); SPADEA, MARIA FRANCESCA, 69115 HEIDELBERG (HD) (DE); MEROLA, ALESSIO, 88100 CATANZARO (CZ) (IT)
(74) Representative: Giuliano, Natalia

(57) **Abstract**

Support device (100) for the dynamic reference sensor for tracking in navigated maxillofacial surgery comprising: - a maxillary plate (101) configured to be anchored to the upper jaw of a patient; - a support (102) configured to house a tracking sensor (103) and connected to the plate (101) by a curved arm (104); characterized in that it is made of biodegradable thermoplastic polymer material.

## Description

The present invention relates to a support device for a dynamic reference sensor for tracking in navigated maxillofacial surgery.

As is known, neuronavigation is a computerized technology that allows the surgeon to know the exact position most of the time during the operation, allowing even deep sites to be reached; allows you to plan the pre-operative phase and set up an image-guided intervention strategy. The patient undergoes preoperative imaging according to a defined protocol and the images are acquired by the neuronavigation device. These images are reprocessed in three dimensions using advanced software, capable of relating the images to the site of the lesion.

Different localization systems are known:
- optical systems, including a group of infrared cameras and retroreflective skin markers on a stably fixed skull, which determine a millimetric correspondence between the magnetic resonance image displayed on the neuronavigator and the exact localization of the superficial and deep brain structures;
- magnetic systems that do not require tight attachment of the head to the patient's bed and can also be applied in the pediatric field.

The latter create a low-energy magnetic field around the patient's head to provide a reference frame and determine the location of a pointer in space. A DRF (dynamic reference frame), i.e. a dynamic reference fiducial, is applied to the scalp and identifies the anatomical position. It is composed of three main elements: one or more sensors placed on the instruments, a magnetic field generator and a central unit.

The sensors record the position and orientation of the instruments with respect to the anatomy in real time. The signals are processed by the central unit, integrated with the preoperative images and sent into an output system that the surgeon uses as a map.

Although these systems can be integrated into any instrument, thanks to the size of the sensors, they however suffer from the problem that the resolution is lower than optical systems, precisely due to the spatial distortion due to the magnetic field.

This type of neuronavigator can be used in traumatology, for computer-guided surgery used in the treatment of fractures of the floor of the orbit; in oncology for the resection of tumors that are difficult to attack in the maxillofacial area, such as the pterygoid and infratemporal fossa; in the removal of foreign bodies from the face.

To date, in fact, the tracking sensor ("Dynamic Reference Frame" or DRF) remains in a fixed point in the center of the forehead, so it would be impossible to access the frontal sinuses or anatomical regions that are difficult to access, such as those mentioned above.

The aim of the present invention is to provide a support device for the DRF sensor in navigated maxillofacial surgery, which allows the allocation of the tracking sensor in an anatomical district, specifically in the oral cavity, different from the frontal region, which is ergonomic and which allows exploration anatomical regions of the hard and soft tissues of the face that are difficult to access, such as the upper and middle third of the face, therefore with characteristics that overcome the limits of current known tracking devices for navigated maxillofacial surgery.

According to the present invention, a support device is created for the dynamic reference sensor for tracking in navigated maxillofacial surgery, as defined in claim 1.

For a better understanding of the present invention, a preferred embodiment is now described, purely by way of nonlimiting example, with reference to the attached drawings, in which:

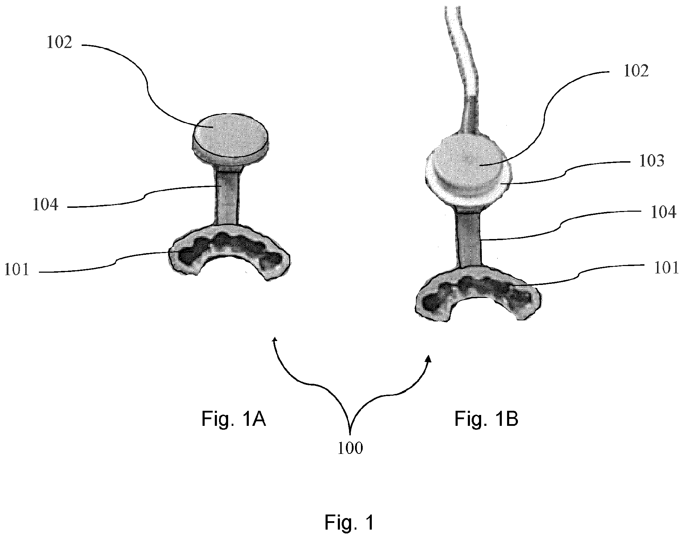

- Figures 1A-1B show three-dimensional views of a support device for the dynamic reference sensor for tracking in navigated maxillofacial surgery, according to the invention;

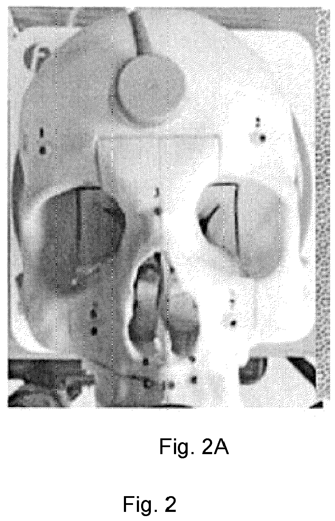

- Figure 2A shows the three-dimensional view of a skull on which the support device for the dynamic reference sensor for tracking in navigated maxillofacial surgery is applied in the position currently used, according to the invention;

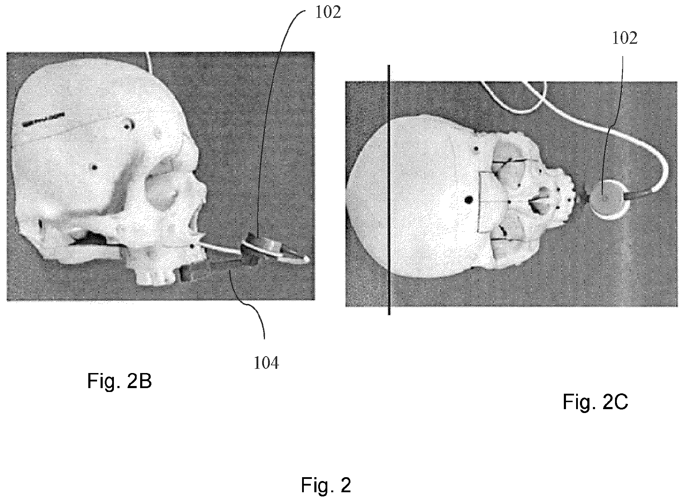

- Figures 2B-2C show three-dimensional views of a skull on which the support device for the dynamic reference sensor positioned in the oral cavity is applied, for tracing in navigated maxillofacial surgery of the temporal, maxillary and oral cavity regions, according to the invention.

With reference to these figures, a support device for the dynamic reference sensor for tracking in navigated maxillofacial surgery is shown, according to the invention.

The support device 100 for the dynamic reference sensor for tracking in navigated maxillofacial surgery includes a plate 101 configured to be anchored to the upper jaw of a patient, a shelf support 102 configured to house a tracking sensor 103 and connected to the plate 101 by a curved arm 104.

Advantageously according to the invention, the device 100 was made by 3D printing using an additive polylactic acid (PLA) filament with a thickness between 1.5 and 2.85 mm and a printing temperature between 190°C and 210°C .

The filament used is a plant-based plastic material (non-ferromagnetic), which commonly uses corn starch as a raw material. This material is a thermoplastic aliphatic polyester and is the main natural raw material used in 3D printing. PLA is a completely biodegradable thermoplastic polymer made from renewable raw materials. Widely used for 3D printing since compared to other filaments it does not release odors during printing.

The base resin, the main constituent of the material, is to be considered biodegradable in accordance with the standards EN ISO 14851:2004 (Replaced by: EN ISO 14851:2019) and/or EN ISO 14852:2004 (Replaced by: EN ISO 14852:2018 ).

Other types of filaments can also be used but we have chosen this one both for the fact that it is biodegradable and biocompatible also for its subsequent use in vivo.

Advantageously according to the invention, the support device 100 is absolutely reliable, and this is demonstrated by the Applicant through tests which are reproducible and from which it appears that the support device 100 can be considered as accurate as traditional locators.

The advantages are notable in terms of surgical precision and minimal invasiveness. In the experiments carried out by the Applicant, the sensor mounted on this device proved to be slightly superior in terms of precision and accuracy in areas such as the middle third of the face. Further advantageously according to the invention, the shelf support 102, which houses the sensor, and the plate 101 are connected to each other by a curved arm of a design suitable not to interfere with the operating procedures and to minimize discomfort for the patient. The shelf support 102 has been optimized on the morphology and mechanical tolerances of a skull model to minimize clearances and guarantee stability under mechanical pressures during surgery. This check was necessary because the sensor, during navigation, must be immune to small movements to ensure adequate accuracy.

In particular, the support device 100 is customized and is therefore created on a traditional dental impression or with a digital method, i.e. with CAD (Computer-Aided Design) or CAM (Computer-Aided Manufacturing) technology. Digital technology uses an intraoral scanner which, through a high-precision optical system, "reads" the oral tissues (teeth and gums) and allows obtaining a 3D image of the complete dental arch.

In particular, the maxillary plate 101 substantially has the shape of a circumferential arc, corresponding to the dental arch of a patient or a portion thereof, and includes on at least one of its surfaces, upper and/or lower, a plurality of concavities corresponding to the dental impression of the patient himself.

Therefore, the support device for the dynamic reference sensor for tracking in navigated maxillofacial surgery according to the invention allows the sensor to be positioned in such a way that the spherical lead references in correspondence with the temporal bones are localized with a lower error, conceivable of 20 -30%) while those near the alveolar process of the maxillary bones are localized with an error comparable to that assessed with the sensor placed on the forehead.

Another advantage of the dynamic reference support device for tracking in navigated maxillofacial surgery according to the invention is, as already underlined, the possibility of exploring even complex regions of the oro-maxillofacial district such as the temporal and infratemporal region in computer surgical navigation -assisted.

Furthermore, the support device for the dynamic reference sensor for tracking in navigated maxillofacial surgery according to the invention is precise and reliable.

Finally, it is clear that modifications and variations can be made to the support device for the dynamic reference sensor for tracking in navigated maxillofacial surgery according to the invention here described and illustrated, without departing from the protective scope of the present invention, as defined in the attached claims.

## Claims

1. Support device (100) for the dynamic reference sensor for tracking in navigated maxillofacial surgery comprising: - a maxillary plate (101) configured to be anchored to the upper maxilla of a patient; - a support (102) configured to house a tracking sensor (103) and connected to the plate (101) by a curved arm (104); **characterized in that** it is made of biodegradable thermoplastic polymer material.

2. Support device (100) according to claim 1, **characterized in that** it is made of biodegradable thermoplastic polymeric material.

3. Support device (100) according to claim 1, **characterized in that** it is made by 3D printing using an additive polylactic acid (PLA) filament.

4. Support device (100) according to claim 3, **characterized in that** said additive polylactic acid (PLA) filament has a thickness between 1.5 and 2.85 mm, subjected to a printing temperature between 190° C and 210°C.

5. Support device (100) according to claim 1, **characterized in that** the support (102) is shelf-like.
